# EUROPÄISCHE PATENTSCHRIFT

(11) **EP 2 060 569 B1**
(45) Veröffentlichungstag und Bekanntmachung des Hinweises auf die Patenterteilung: **19.10.2011**
(21) Anmeldenummer: 09153155.8
(22) Anmeldetag: 24.08.2004
(51) Int. Cl.: C07D 401/12, A61K 31/4439, A61P 7/02

(54) **3-[(2-{[4-Hexyloxycarbonylamino-imino-methyl)-phenylamino]-methyl}-1-methyl-1H-benzimidazol-5-carbonyl)-pyridin-2-yl-amino]-propionsäure-ethylester-Methansulfonat-Hemihydrat und dessen Verwendung als Arzneimittel**
Ethyl 3-[(2-{[4-Hexyloxycarbonylamino-imino-methyl)-phenylamino]-methyl}-1-methyl-1H-benzimidazol-5-carbonyl)-pyridin-2-yl-amino]-propionate methanesulfonate hemihydrate and its use as a medicament
Hemihydrate de méthanesulfonate d'éthylester d'acide 3-[(2-{[4-hexyloxycarbonylamino-imino-methyl)-phenylamino]-methyl}-1-methyl-1H-benzimidazol-5-carbonyl)-pyridin-2-yl-amino]-propionique et son utilisation comme médicament

(30) Priorität: 29.08.2003 DE 10339862
(43) Veröffentlichungstag der Anmeldung: 20.05.2009
(62) Teilanmeldung aus: 04764411.7
(73) Patentinhaber: Boehringer Ingelheim Pharma GmbH & Co. KG, 55216 Ingelheim am Rhein (DE); Boehringer Ingelheim International GmbH, 55216 Ingelheim am Rhein (DE)
(72) Erfinder: Sieger, Peter, 88441, Mittelbiberach (DE); Sobotta, Rainer, 55218, Ingelheim (DE); Schmid, Rolf, 88487, Baltringen (DE)
(74) Vertreter: Hammann, Heinz

(56) Entgegenhaltungen:
- WO-A-03/074056
- HAUEL N H ET AL: "STRUCTURE-BASED DESIGN OF NOVEL POTENT NONPEPTIDE THROMBIN INHIBITORS" JOURNAL OF MEDICINAL CHEMISTRY, AMERICAN CHEMICAL SOCIETY. WASHINGTON, US, Bd. 45, Nr. 9, 2002, Seiten 1757-1766, XP001098844 ISSN: 0022-2623
- MUNGALL D: "BIBR-1048 BOEHRINGER INGELHEIM" CURRENT OPINION IN INVESTIGATIONAL DRUGS, PHARMAPRESS, US, Bd. 3, Nr. 6, Juni 2002 (2002-06), Seiten 905-907, XP001147306 ISSN: 1472-4472
- COLLINS BEN ET AL: "Antithrombotic drug market." NATURE REVIEWS. DRUG DISCOVERY JAN 2003, Bd. 2, Nr. 1, Januar 2003 (2003-01), Seiten 11-12, XP002521015 ISSN: 1474-1776
- CAIRA M R: "CRYSTALLINE POLYMORPHISM OF ORGANIC COMPOUNDS" TOPICS IN CURRENT CHEMISTRY, SPRINGER, BERLIN, DE, Bd. 198, 1998, Seiten 163-208, XP001156954 ISSN: 0340-1022

## Beschreibung

Gegenstand der vorliegenden Erfindung ist die Verbindung 3-[(2-{[4-(Hexyloxy-carbonylamino-imino-methyl)-phenylamino]-methyl}-1-methyl-1*H*-benzimidazol-5-carbonyl)-pyridin-2-yl-amino]-propionsäure-ethylester - Methansulfonat der Formel A und deren Verwendung als Arzneimittel.

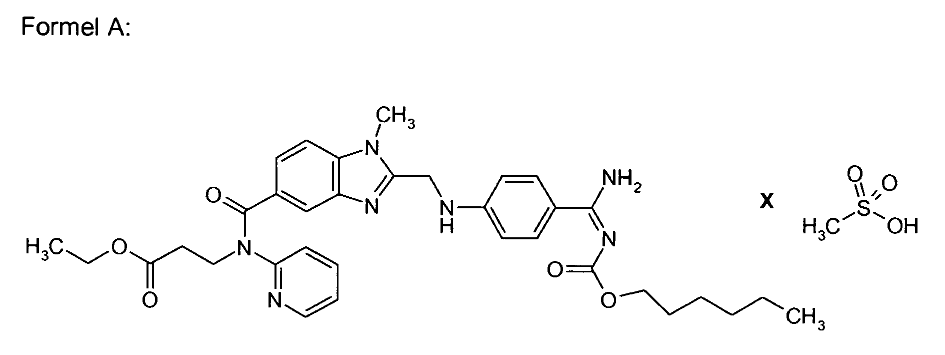

Die Base der Verbindung der Formel A ist bereits aus der WO 98/37075, in der Verbindungen mit einer Thrombin-hemmenden und die Thrombinzeit verlängernden Wirkung offenbart werden, unter dem Namen 1-Methyl-2-[*N*-[4-(*N*-n-hexyloxy-carbonylamidino)phenyl]-amino-methyl]-benzimidazol-5-yl-carbonsäure-*N*-(2-pyridyl)-*N*-(2-ethoxycarbonylethyl)-amid bekannt. Bei der Verbindung der Formel 1 handelt es sich um ein Doppel-Prodrug der Verbindung 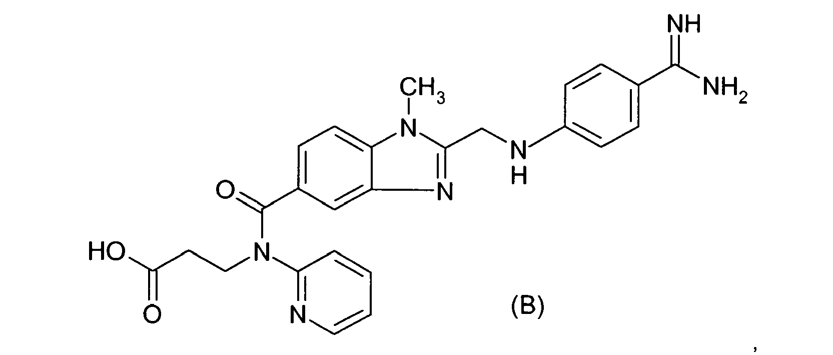 d.h. die Verbindung der Formel A (BIBR 1048 MS) wird erst im Körper in die eigentlich wirksame Verbindung, nämlich die Verbindung der Formel B, umgewandelt. Hauptindikationsgebiete der Verbindung der chemischen Formel A sind die post-operative Prophylaxe von tiefen Venenthrombosen und die Prophylaxe von Schlaganfällen.

Die vorstehend genannten pharmakologisch wertvollen Eigenschaften der im Stand der Technik offenbarten disubstituierten bicyclischen Heterocyclen stellen die Grundvoraussetzung für eine wirksame Verwendung der Verbindungen als Arzneimittel dar. Ein Wirkstoff muß allerdings noch weiteren Anforderungen gerecht werden, um als Arzneimittel zum Einsatz gelangen zu können. Diese Parameter sind zu einem großen Teil mit der physikochemischen Beschaffenheit des Wirkstoffs verbunden.

Ohne Einschränkung darauf sind Beispiele dieser Parameter die Wirkstabilität des Ausgangsstoffes unter verschiedenen Umgebungsbedingungen, die Stabilität im Verlauf der Herstellung der pharmazeutischen Formulierung sowie die Stabilität in den Endzusammensetzungen des Arzneimittels. Der zur Herstellung der Arzneimittelzusammensetzungen verwendete Arzneiwirkstoff sollte daher eine hohe Stabilität aufweisen, die auch unter verschiedenen Umgebungsbedingungen gewährleistet sein muß. Dies ist zwingend erforderlich, um zu verhindern, dass Arzneimittelzusammensetzungen Verwendung finden, in denen neben tatsächlichem Wirkstoff beispielsweise Abbauprodukte desselben enthalten sind. In einem solchen Fall könnte ein in pharmazeutischen Formulierungen vorgefundener Gehalt an Wirkstoff niedriger sein als spezifiziert.

Die Absorption von Feuchtigkeit vermindert den Gehalt an Arzneiwirkstoff wegen der durch die Wasseraufnahme verursachten Gewichtszunahme. Zur Aufnahme von Feuchtigkeit neigende Arzneimittel müssen während der Lagerung vor Feuchtigkeit geschützt werden, beispielsweise durch Zusatz von geeigneten Trockenmitteln oder durch Lagerung des Arzneimittels in einer vor Feuchtigkeit geschützten Umgebung. Zudem kann die Aufnahme von Feuchtigkeit den Gehalt an Arzneiwirkstoff während der Herstellung vermindern, wenn das Arzneimittel der Umgebung ohne jeglichen Schutz vor Feuchtigkeit ausgesetzt wird. Vorzugsweise sollte ein Arzneimittelwirkstoff daher nur in geringem Maße hygroskopisch sein.

Da die Kristallmodifikation eines Wirkstoffs für den reproduzierbaren Wirkstoffgehalt einer Darreichungsform von Bedeutung ist, besteht die Notwendigkeit, eventuell existierenden Polymorphismus eines kristallin vorliegenden Wirkstoffs bestmöglich aufzuklären. Sofern verschiedene polymorphe Modifikationen eines Wirkstoffs auftreten, sollte gewährleistet sein, dass sich die kristalline Modifikation der Substanz in der späteren Arzneimittelzubereitung nicht verändert. Andernfalls könnte dies die reproduzierbare Wirksamkeit des Medikaments nachteilig beeinflussen. Bevorzugt sind vor diesem Hintergrund Wirkstoffe, die nur durch geringen Polymorphismus gekennzeichnet sind.

Ein weiteres Kriterium, welches je nach Wahl der Formulierung oder nach Wahl des Herstellungsverfahrens der Formulierung von unter Umständen herausragender Bedeutung ist, ist die Löslichkeit des Wirkstoffs. Werden beispielsweise Arzneimittellösungen (etwa für Infusionen) bereitgestellt, so ist eine ausreichende Löslichkeit des Wirkstoffs in physiologisch verträglichen Lösemitteln unverzichtbar. Auch für oral zu applizierende Arzneimittel ist eine ausreichende Löslichkeit des Wirkstoffs von großer Wichtigkeit.

Der vorliegenden Erfindung liegt die Aufgabe zugrunde, einen Arzneimittelwirkstoff bereitzustellen, der nicht nur durch eine hohe pharmakologische Wirksamkeit gekennzeichnet ist, sondern ferner den vorstehend genannten physikochemischen Anforderungen bestmöglich gerecht wird.

### Detaillierte Beschreibung der Erfindung

Die vorstehend genannte Aufgabe wird durch das Salz 3-[(2-{[4-(Hexyloxycarbonyl-amino-imino-methyl)-phenylamino]-methyl}-1-methyl-1*H*-benzimidazol-5-carbonyl)-pyridin-2-yl-amino]-propionsäure-ethylester - Methansulfonat der Formel A gelöst.

Unter bestimmten Synthesebedingungen kann man, wie sie beispielsweise in Beispiel 5 beschrieben sind, eine Hydratform erhalten werden, deren Wassergehalt auf ein Hemihydrat schließen läßt.

Für die Arzneimittelanwendung ist es erforderlich, dass der darin enthaltene Wirkstoff in einer einheitlichen Kristallmodifikation vorliegt, damit eine zuverlässige Bioverfügbarkeit gewährleistet ist.

Das erfindungsgemäße Methansulfonat zeichnet sich durch gute Kristallinität und eine geringe Amorphisierung beim Vermahlen und Verpressen aus. Es ist zudem nicht hygroskopisch und in physiologisch verträglichen sauren wäßrigen Medien sehr gut löslich.

Die kristalline Form des erfindungsgemäßen Methansulfonats der Verbindung 3-[(2-{[4-(Hexyloxycarbonylamino-imino-methyl)-phenylamino]-methyl}-1-methyl-1*H-*benzimidazol-5-carbonyl)-pyridin-2-yl-amino]-propionsäure-ethylester ist gekennzeichnet durch einen Schmelzpunkt von T_{Smp.} = 120 ± 5°C (Hemihydrat) (bestimmt über DSC = Differential Scanning Calorimetry; Auswertung über Peak-Maximum; Heizrate: 10°C/min). Die aufgeführten Werte wurden mittels eines DSC 821^{e} der Firma Mettler Toledo ermittelt.

Ein erster Gegenstand der vorliegenden Erfindung ist daher die oben erwähnte polymorphe Form des Salzes 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester - Methansulfonat, bevorzugt in kristalliner Form, gekennzeichnet durch Schmelzpunkte von T_{Smp}. = 120 ± 5°C (bestimmt über DSC; Auswertung über Peak-Maximum; Heizrate: 10°C/min).

Ein weiterer Erfindungsgegenstand sind die Verfahren zur selektiven Herstellung der polymorphen Form.

Erfindungsgemäß erhält man BIBR 1048 MS Hemihydrat, indem man
a) eine Lösung von BIBR 1048 Base in einer Mischung aus 90%-igem wässrigen Ethanol und Essigsäureethylester im Volumen-Verhältnis von ca. 2:5 bei einer Temperatur von ca. 35 °C bis 40 °C langsam mit einer Lösung von einem Äquivalent Methansulfonsäure in Essigsäureethylester versetzt,
b) gegebenenfalls bei Beginn der Auskristallisation des Produkts weiteren Essigsäureethylester zu Verdünnung zugibt,
c) noch ca. 30 Minuten lang bei ca. 35 °C bis 40 °C rührt,
d) dann weitere 30 Minuten lang bei Raumtemperatur rührt,
e) den Niederschlag aus BIBR 1048 MS Hemihydrat absaugt und
f) bei ca. 40 °C im Umluft-Trockenschrank trocknet.

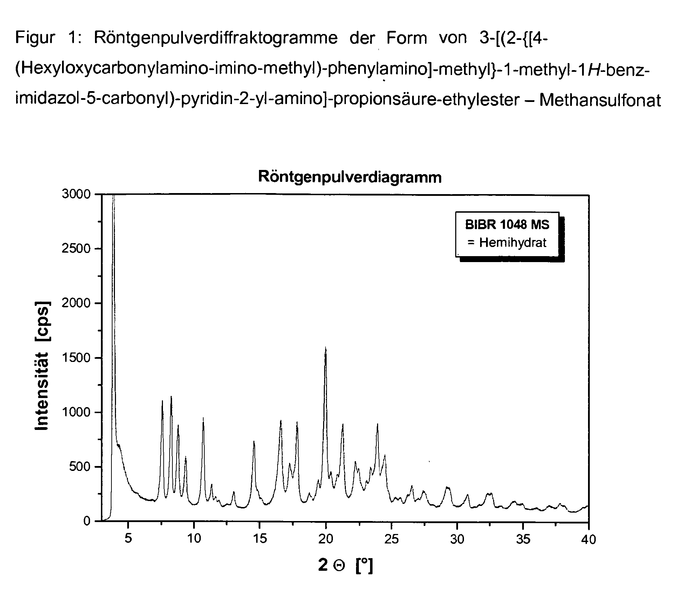

Die erfindungsgemäße kristalline Form des 3-[(2-{[4-(Hexyloxycarbonylaminoimino-methyl)-phenylamino]-methyl}-1-methyl-1*H*-benzimidazol-5-carbonyl)-pyridin-2-yl-amino]-propionsäure-ethylester - Methansulfonats wurde mittels Röntgenpulverbeugung näher untersucht. Das erhaltene Diagramm ist in Figur 1 dargestellt.

Die nachstehende Tabellen 1 führt die bei dieser Analyse erhaltenen Daten auf:

**Tabelle 1: Röntgenpulverreflexe und Intensitäten (normiert) des 3-[(2-{[4-(Hexyloxycarbonylamino-imino-methyl)-phenylamino]-methyl}-1-methyl-1H-benzimidazol-5-carbonyl)-pyridin-2-yl-amino]-propionsäure-ethylester - Methansulfonats (Hemihydrat)**

| **2 Θ [°]** | dₕₖₗ-**Wert [Å]** | **Intensität [%]** |
|---|---|---|
| 3,9 | 22,8 | 100 |
| 4,4 | 20,1 | 10 |
| 5,64 | 15,7 | 2 |
| 7,57 | 11,8 | 16 |
| 8,25 | 10,7 | 17 |
| 8,77 | 10,1 | 12 |
| 9,34 | 9,46 | 7 |
| 10,69 | 8,27 | 13 |
| 11,33 | 7,80 | 3 |
| 11,66 | 7,58 | 1 |
| 11,96 | 7,39 | 1 |
| 13,04 | 6,78 | 3 |
| 14,54 | 6,09 | 11 |
| 15,16 | 5,84 | 1 |
| 16,56 | 5,35 | 13 |
| 17,27 | 5,13 | 6 |
| 17,78 | 4,98 | 12 |
| 18,75 | 4,73 | 1 |
| 19,41 | 4,57 | 3 |
| 19,95 | 4,45 | 24 |
| 20,38 | 4,35 | 4 |
| 20,84 | 4,26 | 4 |
| 21,21 | 4,19 | 12 |
| 22,22 | 4,00 | 6 |
| 22,46 | 3,96 | 5 |
| 23,05 | 3,85 | 3 |
| 23,40 | 3,80 | 4 |
| 23,85 | 3,73 | 12 |
| 24,44 | 3,64 | 7 |
| 25,30 | 3,52 | 1 |
| 25,63 | 3,47 | 1 |
| 26,22 | 3,40 | 2 |
| 26,52 | 3,36 | 3 |
| 27,06 | 3,29 | 1 |
| 27,45 | 3,25 | 2 |
| 29,27 | 3,05 | 3 |
| 30,78 | 2,90 | 2 |
| 32,32 | 2,77 | 2 |
| 32,59 | 2,75 | 2 |
| 34,31 | 2,61 | 1 |
| 34,91 | 2,57 | 1 |
| 36,04 | 2,49 | 1 |
| 37,00 | 2,43 | 1 |
| 37,84 | 2,38 | 1 |
| 38,13 | 2,36 | 1 |

In der voranstehenden Tabelle 1 steht der Wert "2 Θ [°]" für den Beugungswinkel in Grad und der Wert "dₕₖₗ [Å]" für die bestimmten Abstände in Å zwischen den Gitterebenen.

Die Röntgenpulverdiagramme wurden im Rahmen der vorliegenden Erfindung mittels eines Bruker D8 Advanced - Diffraktometers, ausgerüstet mit einem ortsempfindlichen Detektor (OED) und einer Cu-Anode als Röntgenquelle (CuK_{α} - Strahlung, λ = 1.54056 Å, 40 kV, 40 mA) aufgenommen.

Das erfindungsgemäße Hydrat der Verbindung 3-[(2-{[4-(Hexyloxycarbonylaminoimino-methyl)-phenylamino]-methyl}-1-methyl-1*H*-benzimidazol-5-carbonyl)-pyridin-2-yl-amino]-propionsäure-ethylester -Methansulfonat liegt unter Normbedingungen in Form des Hemihydrats vor, aus dem bei einer Temperatur von etwa 120°C, parallel zum Aufschmelzen dieser Form, Wasser entweicht.

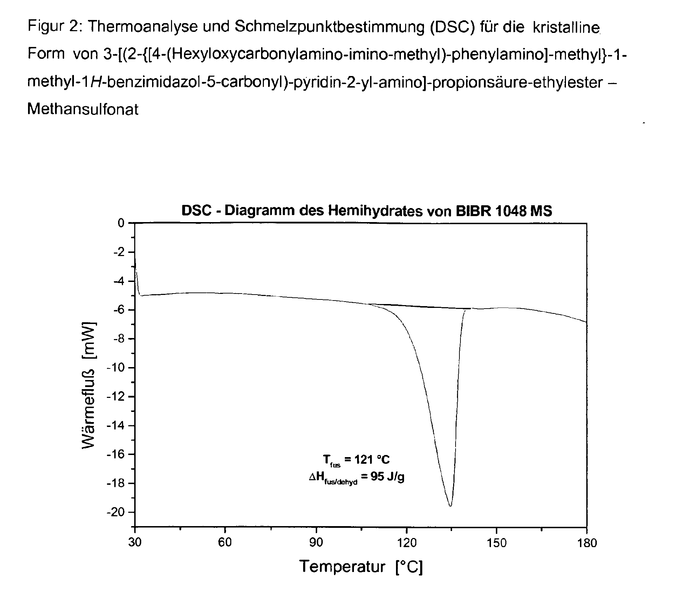

Figur 2 gibt die Thermoanalyse der Form wieder.

### Experimenteller Teil

### Beispiel 5

### 3-[(2-{[4-(Hexyloxycarbonylamino-imino-methyl)-phenylamino]-methyl}-1-methyl-1H-benzimidazol-5-carbonyl)-pyridin-2-yl-amino]-propionsäure-ethylester - Methansulfonat-Hemihydrat

Zu einer Lösung von 10,0 g (15.93 mMol) 3-[(2-{[4-(Hexyloxycarbonylamino-iminomethyl)-phenylamino]-methyl}-1-methyl-1*H*-benzimidazol-5-carbonyl)-pyridin-2-yl-amino]-propionsäure-ethylester - Base (wie in der WO 98/37075 beschrieben hergestellt) in 16,5 ml 90%igem wässrigem Ethanol und 40 ml Essigsäureethylester wurde eine Lösung von 1,53 g (15.93 mMol) Methansulfonsäure in 15 ml Essigsäureethylester unter Rühren bei 35-40°C tropfenweise zugegeben. Nach wenigen Minuten begann das Produkt auszukristallisieren und es wurde mit 30 ml Essigsäureethylester verdünnt. Es wurde noch 30 Minuten lang bei 35-40°C und weitere 30 Minuten bei Raumtemperatur gerührt, dann der Niederschlag abgesaugt, mit ca. 20 ml Essigsäureethylester gewaschen und bei 40°C im Umluft-Trockenschrank getrocknet.
Ausbeute: 99% der Theorie

### Kurzbeschreibung der Abbildungen

Figur 1 zeigt das Röntgenpulverdiffraktogramm der kristallinen Form von 3-[(2-{[4-(Hexyloxycarbonylamino-imino-methyl)-phenylamino]-methyl}-1-methyl-1*H-*benzimidazol-5-carbonyl)-pyridin-2-yl-amino]-propionsäure-ethylester - Methansulfonat.
Figur 2 zeigt die Thermoanalyse und Schmelzpunktbestimmung (DSC) für die kristalle Form von 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester - Methansulfonat.

## Patentansprüche

1. 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester-Methansulfonat in kristalliner Form, **gekennzeichnet durch** einen Schmelzpunkt von T_{Smp}. = 120 ± 5°C (Hemihydrat) (bestimmt über DSC; Auswertung über Peak-Maximum; Heizrate: 10°C/min).

2. Arzneimittel, enthaltend das Salz 3-[(2-{[4-(Hexyloxycarbonylamino-iminomethyl)-phenylamino]-methyl}-1-methyl-1*H*-benzimidazol-5-carbonyl)-pyridin-2-yl-amino]-propionsäure-ethylester-Methansulfonat gemäß Anspruch 1 neben gegebenenfalls einem oder mehreren inerten Trägerstoffen und/oder Verdünnungsmitteln.

3. Verwendung von 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester-Methansulfonat gemäß Anspruch 1 zur Herstellung eines Arzneimittels, welches zur postoperativen Prophylaxe von tiefen Venenthrombosen und zur Prophylaxe von Schlaganfällen geeignet ist.

4. Verfahren zur Herstellung eines Arzneimittels gemäß Anspruch 2, **dadurch gekennzeichnet, dass** auf nichtchemischem Wege das Salz 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester-Methansulfonat gemäß Anspruch 1 in einen oder mehrere inerte Trägerstoffe und/oder Verdünnungsmittel eingearbeitet wird.

5. Verfahren zur Herstellung von 3-[(2-{[4-(Hexyloxycarbonlamino-imino-methyl)-phenylamino]-methyl}-1- methyl-1*H*-benzimidazol-5-carbonyl)-pyridin-2-yl-amino]-propionsäure-ethylester- Methansulfonat Hemihydrat, **dadurch gekennzeichnet, dass** man
a) eine Lösung von 1-Methyl-2-[*N*-[4-(N-n-hexyloxycarbonylamidino)phenyl]-amino-methyl]-benzimidazol-5-yl-carbonsäure-*N*-(2-pyridyl)-*N*-(2-ethoxycarbonylethyl)-amid in einer Mischung aus 90%-igem wässrigen Ethanol und Essigsäureethylester im Volumen-Verhältnis von ca. 2:5 bei einer Temperatur von ca. 35 °C bis 40 °C langsam mit einer Lösung von einem Äquivalent Methansulfonsäure in Essigsäureethylester versetzt,
b) gegebenenfalls bei Beginn der Auskristallisation des Produkts weiteren Essigsäureethylester zu Verdünnung zugibt,
c) noch ca. 30 Minuten lang bei ca. 35 °C bis 40 °C rührt,
d) dann weitere 30 Minuten lang bei Raumtemperatur rührt,
e) den Niederschlag aus 3-[(2-{[4-(Hexyloxycarbonlamino-imino-methyl)-phenylamino]-methyl}-1- methyl-1*H*-benzimidazol-5-carbonyl)-pyridin-2-yl-amino]-propionsäure-ethylester- Methansulfonat Hemihydrat absaugt und
f) bei ca. 40 °C im Umluft-Trockenschrank trocknet.

## Claims

1. Ethyl 3-[(2-{[4-(hexyloxycarbonylamino-imino-methyl)-phenylamino]-methyl}-1-methyl-1H-benzimidazole-5-carbonyl)-pyridin-2-yl-amino]-propionate-methanesulphonate in crystalline form, **characterised by** a melting point of T_{m.p..}= 120 ± 5°C (hemihydrate) (determined by DSC; evaluation by peak maximum; heating rate: 10°C/min).

2. Medicament, containing the salt ethyl 3-[(2-{[4-(hexyloxycarbonylaminoimino-methyl)-phenylamino]-methyl}-1-methyl-1H-benzimidazole-5-carbonyl)-pyridin-2-yl-amino]-propionate-methanesulphonate according to claim 1, optionally together with one or more inert carriers and/or diluents.

3. Use of ethyl 3-[(2-{[4-(hexyloxycarbonylamino-imino-methyl)-phenylamino]-methyl}-1-methyl-1H-benzimidazole-5-carbonyl)-pyridin-2-yl-amino]-propionate-methanesulphonate according to claim 1 for preparing a medicament which is suitable for the post-operative prophylaxis of deep vein thrombosis and the prevention of stroke.

4. Process for preparing a medicament according to claim 2, **characterised in that** by a non-chemical method the salt ethyl 3-[(2-{[4-(hoxyloxycarbonylaminoimino-methyl)-phenylamino]-methyl}-1-methyl-1H-benzimidazole-5-carbonyl)-pyridin-2-yl-amino]-propionate-methanesulphonate according to claim 1 is incorporated in one or more inert carriers and/or diluents.

5. Process for preparing ethyl 3-[(2-{[4-(hexyloxycarbonylamine-imino-methyl)-phenylamino]-methyl}-1-methyl-1*H*-benzimidazole-5-carbonyl)-pyridin-2-yl-amino]-propionate-methanesulphonate hemihydrate, **characterised in that**
a) a solution of one equivalent of methanesulphonic acid in ethyl acetate is slowly added to a solution of 1-methyl-2-[N-[4-(N-n-hexyloxycarbonylamidino)-phenyl]-amino-methyl]-benzimidazol-5-yl-carboxylic acid-N-(2-pyridyl)-N-(2-ethoxycarbonylethyl)-amide in a mixture of 90% aqueous ethanol and ethyl acetate in a ratio by volume of approx. 2:5 at a temperature of approx. 35°C to 40°C,
b) optionally, as the product begins to crystallise out, further ethyl acetate is added for dilution,
c) the mixture is stirred for about another 30 minutes at approx. 35°C to 40°C,
d) then stirred for a further 30 minutes at ambient temperature,
e) the precipitate of ethyl 3-[(2-{[4-(hexyloxycarbonylamino-imino-methyl)-phenylamino]-methyl}-1-methyl-1*H*-benzimidazole-5-carbonyl)-pyridin-2-yl-amino]-propionate-methanesulphonate hemihydrate is suction filtered and
f) dried at approx. 40°C in the circulating air drying cupboard.

## Revendications

1. Méthane-sulfonate d'éthylester d'acide 3-[(2-{[4-(hexyloxycarbonylamino-imino-méthyl)-phénylamino]-méthyl}-1-méthyl-1H-benzimidazol-5-carbonyl)-pyridin-2-yl-amino]-propionique sous forme cristalline, **caractérisé par** un point de fusion T_{Smp} = 120 ± 5°c (hémihydrate) (déterminé par OSC ; évaluation par pic maximal, vitesse de chauffage : 10°C/min).

2. Médicament contenant le sel méthane-sulfonate d'éthylester d'acide 3-[(2-{[4-(hexyloxycarbonylamino-imino-méthyl)-phénylamino]-mêthyl}-1-méthyl-1H-benzimidazol-5-carbonyl)-pyridin-2-ylamino]-propionique selon la revendication 1, parallèlement, éventuellement, à un ou plusieurs véhicules et/ou diluants inertes.

3. Utilisation de méthane-sulfonate d'éthylester d'acide 3-[(2-{[4-(hexyloxycarbonylamino-imino-méthyl)-phénylamino]-méthyl}-1-méthyl-1H-benzimidazol-5-carbonyl)-pyridin-2-yl-amino]-propionique selon la revendication 1, pour la production d'un médicament qui est approprié pour la prophylaxie post-opératoire de la thrombose veineuse profonde et pour la prophylaxie des AVC.

4. Procédé de production d'un médicament selon la revendication 2, **caractérisé en ce que**, de façon non chimique, le sel de méthane-sulfonate d'éthylester d'acide 3-[(2-{[4-(hexyloxycarbonylamino-imino-méthyl)-phénylamino]-méthyl}-1-méthyl-1H-benzimidazol-5-carbonyl)-pyridin-2-yl-amino]-propionique selon la revendication 1 est introduit dans un ou plusieurs véhicules et/ou solvants inertes.

5. Procédé de production d'hémihydrate de méthane-sulfonate d'éthylester d'acide 3-[(2-{[4-(hexyloxycarbonylamino-imino-mêthyl)-phénylamxno]-méthyl}-1-méthyl-1H-benzimidazol-5-carbonyl-pyràdin-2-yl-amino)-propionique, **caractérisé en ce que**
a) on mélange lentement une solution de N-(2-pyridyl)-N-(2-éthoxycarbonyléthyl)-amide d'acide (1-methyl-2-[N-[4-(N-n-hexyloxycarbony-amino)-phényl]-amino-méthyl]-benzimidazol-5-yl-carboxylique dans un mélange d'éthanol aqueux à 90 % et d'éthylester d'acide acétique en un rapport en volume d'environ 2:5 à une température d'environ 35°C à 40°C avec une solution d'un équivalent d'acide méthanesulforique dans de l'éthylester d'acide acétique,
b) éventuellement, au début de la cristallisation du produit, on ajoute encore de l'éthylester d'acide acétique pour dilution,
c) on mélange pendant encore environ 30 minutes à environ 35°C à 40°C,
d) puis on agite pendant encore 30 minutes supplémentaires à température ambiante,
e) on aspire le précipité d'hémihydrate de méthane-sulfonate d'éthylester d'acide 3-[(2-([4-(hexyloxycarbonylarnino-imino-méthyl)-phénylamino]-méthyl)-1-méthyl-1H-benzimidazol-5-carbonyl) -pyridin-2-yl-amino]-propionique et
f) on le sèche à environ 40°C dans une étuve à circulation d'air.
